Europäisches Patentamt

(19) European Patent Office

Office européen des brevets

(11) Publication number: **0 178 928**

**A2**

(12) **EUROPEAN PATENT APPLICATION**

(21) Application number: **85307484.7**

(51) Int. Cl.⁴: **A 61 K 39/00**

(22) Date of filing: **17.10.85**

(30) Priority: **18.10.84 GB 8426366**

(43) Date of publication of application:
**23.04.86 Bulletin 86/17**

(84) Designated Contracting States:
**AT BE CH DE FR GB IT LI LU NL SE**

(71) Applicant: **GLAXO GROUP LIMITED**
**Clarges House 6-12 Clarges Street**
**London W1Y 8DH(GB)**

(72) Inventor: **Urquhart, George MacDonald**
**28 Queen Street**
**Helensburgh Scotland(GB)**

(72) Inventor: **Mulligan, William**
**Brooks House Cardross**
**Dunbartonshire Scotland(GB)**

(72) Inventor: **McIntyre, William Ian MacKay**
**Stuckenduff Rhu**
**Dunbartonshire Scotland(GB)**

(72) Inventor: **Jennings, Francis William**
**8 North Grange Road**
**Glasgow Scotland(GB)**

(72) Inventor: **Jarrett, William Fleming Hoggan**
**60 Netherblane**
**Blanefield Scotland(GB)**

(72) Inventor: **Bain, Robert Kennedy**
**155 Old Castle Road**
**Glasgow Scotland(GB)**

(74) Representative: **Szczuka, Jan Tymoteusz et al,**
**Cruikshank & Fairweather 19 Royal Exchange Square**
**Glasgow G1 3AE Scotland(GB)**

(54) **Vaccines.**

(57) The present invention provides a vaccine for use against *Dictyocaulus viviparus* infection in cattle which vaccine is a sterile composition comprising live immunogenic attenuated *Dictyocaulus viviparus* third stage larvae.

The present invention also provides a method of sterilising *Dictyocaulus viviparus* larvae as well as the use of sterilants in the sterilisation of these larvae.

EP 0 178 928 A2

VACCINES

The present invention relates to vaccines and in particular to improved vaccines suitable for use in immunizing cattle against infection by Dictyocaulus viviparus (lungworm), a method for preparing the vaccines, and a method for immunizing cattle against Dictyocaulus viviparus using such vaccines.

Although the principle of vaccination in the prevention of diseases caused by bacterial and viral infections is well established, relatively little success has attended the development of vaccines against nematode parasites in general. In spite of intensive work with nematode parasites in many laboratories throughout the world over the past 30 to 40 years very few vaccines have been produced commercially with any success. The reasons for this are not surprising considering the complexity of the host response to nematode parasites and probably include the following:-

(a) The organisms are multicellular and go through various stages of morphological development in the host. This suggests that the immunological response of the host is correspondingly complex and that it is difficult to isolate the protective antigens.

(b) When nematode diseases are acquired by ingestion, immunological barriers are erected in the gastrointestinal tract and associated lymphoid tissue which influences the acquisition of immunity.

(c) Successful vaccination against nematode parasites has depended on the use of living organisms which require to be suitably attenuated i.e. organisms which have been treated in a way, for example by irradiation, which interferes with the normal development and so reduces the pathogenicity but does not destroy the immunogenicity of the organisms. Perhaps the most successful in this category is the oral vaccine used to protect calves against bovine parasitic bronchitis caused by the lungworm Dictyocaulus viviparus. In this vaccine first-stage larvae hatched from ova are cultured until they reach the third or infective stage and are then exposed to 40 Kr X-irradiation. Two doses each containing at least 1000 of the partially inactivated larvae are fed to calves orally i.e. by the natural infection route. The partially inactivated larvae migrate via mesenteric lymph nodes and blood to the lung, where most die and are eliminated at an early stage of development. In the process, an immune response to further challenge is established.

Although the oral vaccine mentioned above containing partially inactivated larvae has been successful, the oral administration route has been found to be somewhat difficult to operate in practice due to the lack of cooperation on the part of the animal which can also result in inaccurate dosing of the vaccine. Thus an injectable vaccine would be advantageous. The only parenterally administered vaccine that has been developed for providing protection against nematode parasites was a vaccine for immunizing dogs against Ancylostoma caninum which was only marketed for a short period. However, the preparation of such a vaccine was facilitated by the ease with which the eggshells of this nematode parasite could be sterilised i.e. made free from microbial contamination before hatching of the first stage larvae and also because the method of administration mimicked one of the natural ways by which this parasite attacks its host i.e. through the skin.

A disincentive in developing parenteral routes of administration for irradiated vaccines in the case of Dictyocaulus viviparus has been the difficulty in obtaining the required third stage larvae in sterile form which, in the case of Dictyocaulus viviparus larvae are recovered from bovine faeces, without seriously affecting their infectivity and longevity.

Normal third stage Dictyocaulus viviparus larvae have been washed and treated with antibiotics such as nystatin and streptomycin. However, such treatments would not effectively sterilise the larvae and indeed, these antibiotics are generally unacceptable in vaccine preparations.

It is an object of the present invention to avoid or minimise one or more of the above disadvantages and in particular to provide a vaccine containing live sterilised *Dictyocaulus viviparus* larvae, which confers a high level of immunity and which is suitable for parenteral administration.

It has now been found that, with suitable treatment, *Dictyocaulus viviparus* larvae collected from faeces can be sterilised using a suitable sterilising agent without significantly affecting their longevity and infectivity and that the radiation-attenuated larvae when given parenterally, preferably subcutaneously, can confer a high level of immunity against *Dictyocaulus viviparus* infections in cattle.

The present invention provides a vaccine for use against *Dictyocaulus viviparus* infection in cattle which vaccine is a sterile composition comprising live immunogenic attenuated *Dictyocaulus viviparus* third stage larvae.

An effective sterilising agent in accordance with the invention is a chemical sterilant which is capable of effectively sterilising the larvae without causing exsheathment or impairment of the viability, infectivity and longevity of the larvae.

For the avoidance of doubt it is noted that the term "sterile" is used herein to denote freedom from microbial contamination and related terms and expressions such as sterilisation, sterilant, and sterilising agent are to be construed in accordance with this meaning unless the contrary is specifically indicated.

Among sterilants which may be used are acids such as dilute hydrochloric acid; oxidising agents such as potassium permanganate and potassium dichromate; protein poisons such as povidone-iodine; and antiseptic agents such as the chloramines, for example chloramine-T (p-toluene sulphon-chloramide) and halogenated phenols and derivatives thereof such as 2,4-dichloro-3, 5-dimethylphenol, 2,2'-thio-bis-4, 6-dichlorophenol, 4-chloro-3-methylphenol, p-chlorophenol, p-bromophenol, 2,4-dichlorophenol, 2,4,5-trichlorophenol and 2,2'-dihydroxy chlorinated diphenylmethanes.

Preferred sterilants of the invention include the 2,2'-dihydroxy chlorinated diphenylmethanes such as 5,5'-dichloro-2,2'-dihydroxydiphenylmethane (dichlorophen commercially available under the Trade Name Panacide), halogenated phenols such as 2,4-dichlorophenol and 4-chloro-3-methylphenol and chloramines such as chloramine-T.

Particularly effective sterilants in accordance with the present invention have been found to be the 2,2'-dihydroxy chlorinated diphenylmethanes, in particular, dichlorophen. Conveniently this compound may be used in the form of an alkali metal salt for example, the monosodium salt, though of course other soluble salts may also be used. In practice, the dichlorophen is employed in the form of an aqueous solution of the monosodium salt with which the larvae are contacted.

The sterilants may be used at various concentrations to achieve sterilisation without substantially reducing viability and infectivity. In practice, concentrations of as low as 0.002% w/v have been found to provide effective sterilisation, but higher concentrations, preferably 0.004% w/v and up to for example 1.2% w/v may also be used without substantially reducing longevity.

The sterilisation may be effected at various temperatures for various periods of time depending upon the nature and concentration of the sterilant used. Conveniently the sterilisation is effected at from $4^{\circ}C$ to $25^{\circ}C$ within a period of at least 1 minute, for example, from 5 minutes to 72 hours, lower temperatures being generally preferred in order to maximise longevity. In general, minimum concentrations of the sterilising agent are preferred in order to reduce or even avoid altogether the need for washing out of the agent from the larvae after sterilisation. Thus, where a relatively low concentration of sterilising agent is used, for example from 0.002 to 0.007% w/v, preferably 0.004% w/v, this does not require to be removed after sterilisation so

that the larvae may simply be kept in the sterilising solution until required for administration to the cattle to be immunised.

The larvae may be attenuated, conveniently after but alternatively before sterilisation by treatment with ionising radiations e.g. X-rays, or $\gamma$-rays (conveniently from a cobalt $^{60}$ source), or by treatment with a radiomimetic substance such as for example triethylene melamine (e.g. 0.7% w/v aqueous solution at $26^{O}C$ for 1 hour). Irradiation is preferred as it avoids the possible need for separation of the larvae from any radiomimetic substance used.

The third stage Dictyocaulus viviparus larvae used in accordance with the present invention may be obtained in any known manner but conveniently are obtained by collecting fresh faeces which contain first stage Dictyocaulus viviparus larvae from infected calves and then incubating the faeces, for example at a temperature of from 10 to $25^{O}C$, conveniently at $18^{O}C$, for a period of time sufficient for the larvae to mature to their third stage. The period of time will vary according to inter alia the incubation temperature, but is generally in the range from 6 to 8 days at $18^{O}C$. The third stage larvae are collected from the faeces by washing and filtration in generally known manner.

After attenuation and sterilisation as described above, the larvae are, if not already in such a medium, washed and suspended in a physiologically acceptable sterile vehicle to provide a vaccine of the invention.

The present invention also extends to a method of preparing a vaccine, which method comprises the steps of sterilising Dictyocaulus viviparus larvae before or after attenuation, without damaging their immunogenicity, and presenting the third stage larvae in a sterile physiologically acceptable vehicle. Preferably attenuation is carried out before sterilisation.

The vaccine of the present invention is preferably administered parenterally, most preferably subcutaneously. Injection may be to any convenient part of the body of the animal. This will usually be a part of the body where there is some loose skin such as on the shoulder or chest wall. In the case of a vaccine of the invention for parenteral administration the larvae are desirably presented in a physiologically acceptable injection vehicle at a concentration of from 500 to 1000 larvae per ml of the solution, preferably about 750 larvae/ml, for injection into cattle to be immunised.

Desirably the larvae are injected at a dosage rate of from 10 to 80 larvae per kg bodyweight, advantageously in two or more doses separated by at least 14 days, preferably at least 21 days for example, 28 days.

Thus, the present invention also extends to a method of vaccination of cattle against Dictyocaulus viviparus infection (lungworm) by parenteral administration, preferably by subcutaneous injection, of an immunogenic dose of a vaccine of the present invention. Preferably the vaccine of the invention is in unit dosage form for example, ampoules containing from 500 to 3000 of the larvae, preferably about 1500 larvae. Conveniently each dose is presented in from 1 to 25 mls preferably from 1 to 5 mls and more preferably about 2 mls of sterile physiologically acceptable injection vehicle.

In addition, the present invention extends to a vaccine of the invention which is in the form of a sterile physiologically acceptable orally ingestible composition, the sterilised and attenuated larvae being presented in a sterile physiologically acceptable oral ingestion vehicle.

In general the vaccine to be administered orally is formulated so that an effective dosage, for example, from 500 to 3000 of the larvae, preferably about 1500 larvae, is presented in a volume of from 5 to 50 mls, preferably 25 mls of sterile physiologically acceptable vehicle. Thus the oral vaccine generally contains the larvae at a concentration in the range from 10 to 600 per ml, preferably about 60 larvae per ml.

In yet another aspect, the present invention provides the use of a sterilant as described above for the sterilisation of Dictyocaulus viviparus larvae without impairment of viability, and in particular the use of 5,5'-dichloro-2,2'-dihydroxydiphenylmethane (dichlorophen) in the form of its monosodium salt in the sterilisation of Dictyocaulus viviparus larvae.

Further preferred features and advantages of the invention will appear from the following detailed examples given by way of illustration only.

Example 1

(a) Recovery and Sterilisation of Dictyocaulus viviparus Larvae.

Third stage Dictyocaulus viviparus larvae were collected from infected cattle faeces and suspended in water (ca. 750 larvae in 1 ml suspension) to which was added aqueous dichlorophen (monosodium salt) (3 ml) to give a final dichlorophen concentration of 0.0024% w/v. After 1 hour the aqueous solution was replaced by 2 changes of sterile water.

(b) Testing of Sterilisation Procedure

Escherichia coli bacteria were isolated from third stage Dictyocaulus viviparus larvae which had been collected from infected cattle faeces and washed in water, and were then sub-cultured. The cultured Escherichia coli were then made up to an Optical Density of 0.90 in sterile water and then added to aqueous dichlorophen (monosodium salt) solutions of varying concentrations in the ratio of 1:1000 and incubated for 1 hour at ambient temperature. The incubated solution was further diluted 1:100 and 0.1 ml of the further diluted solution plated onto agar. The agar plates were incubated at 37°C and inspected for the presence of bacterial colonies after 18 hours.

Results

| Dichlorophen concentration | Number of bacterial colonies |
|---|---|
| 0.4% w/v | 0 |
| 0.24% w/v | 0 |
| 0.024% w/v | 0 |
| 0.0024% w/v | 0 |

| Dichlorophen concentration | Number of bacterial colonies |
|---|---|
| 0% w/v (Control) | 780 |

The above results indicate that <u>Escherichia coli</u> bacteria present in the washed larvae were killed at all four different dichlorophen concentrations employed. Bacterial growth was seen in the Control.

(c) <u>Testing of Sterilisation Procedure</u>

Five boxes each containing 12 bottles each containing 24 mls of oral <u>Dictyocaulus</u> <u>viviparus</u> lungworm vaccine (Dictol – Trade Mark of Glaxo Group Limited) were taken and to each of 10 bottles was added 240 µl of 0.4% w/v dichlorophen (monosodium salt) to give a final concentration of 0.004% w/v dichlorophen. Two bottles of vaccine per box remained as controls. Each box was then treated as follows:

<u>Box 1</u>: To each bottle was added <u>Escherichia coli</u> (N.C.T.C. Accession No. 9002) to a concentration of approximately $10^4$ organisms/ml.

<u>Box 2</u>: To each bottle was added <u>Pseudomonas aeruginosa</u> (N.C.T.C. Accession No. 6750) to a concentration of approximately $10^4$ organisms/ml.

<u>Box 3</u>: To each bottle was added <u>Clostridium perfringens</u> (N.C.T.C. Accession No. 8237) to a concentration of approximately $10^4$ organisms/ml.

<u>Box 4</u>: To each bottle was added <u>Salmonella abony</u> (N.C.T.C. Accession No. 6017) to a concentration of approximately $10^4$ organisms/ml.

<u>Box 5</u>: No extraneous bacteria were added.

All the boxes were stored at room temperature for 6 hours followed by 3 days at $4^\circ$C. At the end of this period the contents of each bottle were filtered on to a cellulose acetate filter and incubated in thioglycollate medium for 7 days.

In all boxes no growth was observed in the dichlorophen treated bottles whereas heavy growth was found in all the Control bottles.

(d) <u>Testing of Sterilisation Procedure</u>

Approximately $10^6$ infectious units of IBR virus (infectious bovine rhinotracheitis virus) were added to a bottle (24 ml) of oral Dictyocaulus viviparus lungworm vaccine (Dictol). The sample was diluted by a factor of 1:2,000 and then dichlorophen (monosodium salt) was added to a final concentration of 0.004% w/v. The sample was then stored at room temperature for 6 hours and $4^O$C for 3 days. At the end of this period no virus could be isolated from the sample.

The above experiment was repeated using mucosal disease virus. Again no virus could be isolated following incubation.

The above results indicate that dichlorophen is effective as a sterilant and the vaccine according to the present invention is sterile and therefore suitable for parenteral administration.

Example 2

Testing of Sterility of Dictyocaulus viviparus Larvae

Third stage Dictyocaulus viviparus larvae were collected from infected cattle faeces and suspended in water (ca. 10,000 larvae per ml suspension) to which was added an equal volume of aqueous dichlorophen (monosodium salt) to obtain a final dichlorophen concentration of 0.0024% w/v. After 1 hour the aqueous dichlorophen solution was replaced by 2 changes of sterile water and again made up to a concentration of ca. 10,000 larvae per ml. Controls were set up using sterile water in place of the dichlorophen solution and also with sterile water alone and selected 1 ml aliquots of the test and control materials subjected to grinding in a 'Griffiths' tissue grinder for 5 minutes until the larvae disintegrated.

All the test and control samples were then diluted 1:10 and 1:100, filtered through microbiological filters (0.22 µm pore size) and the filters then placed on nutrient agar plates and incubated at $37^O$C. The plates were inspected for the presence of bacterial colonies after 18 hours.

Results

| Treatment | No. of bacterial colonies | |
|---|---|---|
| | ( Final dilution ) | |
| | ( 1:10 | 1:100 ) |

Controls

| | | |
|---|---|---|
| Water (alone) | O | O |
| larvae and water | tmtc* | 62 |
| water and grinding | O | O |

Test samples

| | | |
|---|---|---|
| larvae and dichlorophen | O | O |
| larvae and dichlorophen and grinding | O | O |

*tmtc = too many to count.

Example 3

Sterilisation of Dictyocaulus viviparus Larvae and Examination of Infectivity

Six doses of 5,000 third stage Dictyocaulus viviparus larvae were made up. Two were made up to 20 ml with distilled water and used as controls. The remaining four were made up to 20 ml in 0.0024% w/v dichlorophen (monosodium salt). These were left for 90 minutes and then the supernatant was replaced with sterile water 3 times allowing 30 minutes for sedimentation between each wash. All six doses were stored at 4°C until used. The day the doses were made up was taken as day O. Three calves were infected on day 14 and three on day 42 - two with dichlorophen (monosodium salt) treated larvae and one with untreated larvae at each occasion. Each calf was killed 24 days after it was infected and the total number of worms present in the lungs was counted.

Result

| | day 14 infection | day 42 infection |
|---|---|---|
| Dichlorophen (monosodium salt) wash | 1090 | 495 |
| Dichlorophen (monosodium salt) wash | 1405 | 840 |
| Control | 1780 | 645 |

All calves displayed clinical symptoms of husk (lungworm) and at necropsy showed typical lungworm lesions thus demonstrating the infectivity of the larvae.

Example 4

Preparation and Use of Dictyocaulus viviparus Vaccine Third stage Dictyocaulus viviparus larvae were attenuated by irradiation of a suspension of the larvae having a depth of 1 cm, in the beam of an X-ray machine using external filters consisting of 0.25 mm of copper and 1.0 mm of aluminium (half value layer 8 mm of aluminium: approximately 0.2 Kr per minute). Irradiation was continued until a dose of 40 Kr measured at the surface of the suspension had been delivered. The attenuated larvae were then sterilised in accordance with the sterilisation procedure of Example 3. The attenuated and sterilised larvae were then reconstituted in sterile water. Two calves were vaccinated subcutaneously in the right fore leg with two doses of 2400 larvae given four weeks apart. Four weeks after the second dose these two calves plus one control were challenged with infective third stage Dictyocaulus viviparus larvae at a dosage rate of 30 larvae per kg bodyweight of the calf. Each calf was killed 28 days after it was infected and the total number of worms present in the lungs was counted.

Result

| | |
|---|---|
| Experimental calves : | 18 worms at necropsy |
| | 109 worms at necropsy |
| Control Calf : | 1393 worms at necropsy |

The above results correspond to a protection of about 95%.

Example 5

Preparation and Use of Dictyocaulus viviparus Vaccine

The above procedure of Example 4 was repeated with 10 experimental calves and 4 controls but with each dose of vaccine containing only 1,000 of the third stage larvae.

Results

The four control calves contained 514, 946, 1025, and 2250 worms, respectively at necropsy whilst the vaccinated calves contained 0, 0, 2, 2, 9, 34, 38, 150, and 290 worms, respectively, in their lungs at necropsy. The mean numbers of worms were 52.5 in the vaccinates and 1184 in the controls corresponding to a protection of about 95%.

Example 6

Preparation and Use of Oral and Injectable Dictyocaulus
viviparus Vaccines

Dictyocaulus viviparus third-stage larvae were irradiated and rendered sterile by the incorporation of 0.004% w/v dichlorophen (monosodium salt) into the aqueous medium containing the larvae. The resulting vaccine containing the irradiated larvae together with the dichlorophen incorporated therein was administered orally or subcutaneously as described in the following experiment:

Eighteen calves were divided into three groups of six calves.

The calves in Group One were each vaccinated orally with 1500 dichlorophen treated, irradiated larvae on days 0 and 28.

The calves in Group Two were each vaccinated subcutaneously with 1500 dichlorophen treated, irradiated larvae on days 0 and 28.

The calves in Group Three remained unvaccinated and acted as challenge controls.

All three groups of calves were challenged with 3000 normal lungworm larvae on day 56 and killed on days 80 and 81. The worm burdens of the 18 calves are listed in Table 1 and the mean output of lungworm larvae per gramme (lpg) of faeces at post-mortem in each group is shown in Table 2.

Table 1

Post-mortem worm burdens

| Group One (oral vaccination) | Group Two (subcutaneous vaccination) | Group Three (controls) |
|---|---|---|
| 0 | 0 | 864 |
| 0 | 3 | 1062 |
| 0 | 4 | 1140 |
| 16 | 18 | 1149 |
| 100 | 25 | 1275 |
| 130 | 110 | 1655 |
| mean 41 | 26.7 | 1190.8 |
| percentage protection 96.6% | 97.8% | 0% |

## Table 2
### Faecal Larval Output

The mean output of Dictyocaulus viviparus larvae per gramme (lpg) of faeces at post-mortem in each group was as follows:

| Group One (oral vaccination) | 1 lpg |
|---|---|
| Group Two (subcutaneous vaccination) | 0 lpg . |
| Group Three (controls) | 87.4 lpg |

As can be seen from Table 1 the degree of protection was 96.6% in Group One vaccinated orally and 97.8% in Group Two vaccinated subcutaneously. The faecal larval output in these groups, shown in Table 2 was 1 and 0 lpg respectively. Thus, as can be seen from the post-mortem worm burdens and faecal larval output, the two vaccinated groups both showed a considerable degree of protection against challenge.

This example demonstrates the efficacy of the vaccine and the degree of protection against Dictyocaulus viviparus challenge conferred by the sterile vaccine of the invention. The vaccine is equally effective whether administered orally or subcutaneously.

### Example 7
### Sterilisation of Dictyocaulus viviparus Larvae

Sterilants were added to larvae suspensions of Dictyocaulus viviparus third stage larvae and viability assessed by determining the survival rate of the larvae without exsheathment and microscopic examination of the larvae. The sterility of treated larvae was assessed indirectly by determining the bactericidal activity of various sterilants using the following procedure.

A suspension of bacteria was made such that it had an optical density (O.D.) at 600 nm. of 0.92 (approximately $10^9$ bacteria/ml.). This was diluted 1:10 with sterile water then 0.1 ml was added to 9.9 ml of the following solutions: 1%, 0.8% povidone-iodine; 2%, 1%, 0.5%, 0.2% potassium dichromate and sterile water. After 1 hour at room temperature these were diluted 1:100 and 0.1ml was plated on to agar. The following results were obtained:

| Sterilant | Treatment Time (hours) | Sterility (no. of colonies) | Viability (survival rate) |
|---|---|---|---|
| 0.1N HCl at pH2 | 1.5 | - | 100% |
| Povidone-iodine (0.8% w/v) | 3 | - | 90% |
| Povidone-iodine (1% w/v) | 1 | 1 | - |
| Potassium dichromate (2% w/v) | 1 | O | - |
| Potassium dichromate (2% w/v) | 2 | - | 100% |
| Potassium dichromate (1% w/v) | 1 | 2 | - |
| Potassium dichromate (0.5% w/v) | 1 | O | - |
| Potassium dichromate (0.2% w/v) | 1 | 1 | - |
| Water (Control) | 1 | 38 | 100% |

The experiment demonstrates the efficacy of the above-mentioned sterilants for use in accordance with the present invention.

Example 8

Sterilisation of Dictyocaulus viviparus Vaccine

A number of different sterilising agent solutions having various concentrations was prepared as follows:

P-chlorophenol and p-bromophenol (0.5g) were each dissolved in ethanol (4.5ml). In each case 0.1ml of this solution was added to 9.9ml distilled water and 0.1ml of this further diluted solution was added to another 9.9ml distilled water to give solutions with three different concentrations : 10% w/v, 0.1% w/v and 0.001% w/v. 2,4-dichlorophenol and 4-chloro-3-methylphenol (0.5g) were each dissolved in ethanol (4.5ml). In each case 0.1ml of this solution was added to 9.9ml of ethanol and 0.1ml of this further diluted solution added to another 9.9ml of ethanol. Again this gave solutions with three different concentrations: 10% w/v, 0.1% w/v and 0.001% w/v.

Chloramine-T hydrate (0.5g) was dissolved in distilled water (4.5ml) and diluted further in water as above to give solutions with three different concentrations: 10% w/v, 0.1% w/v and 0.001% w/v. Ethanol controls containing corresponding amounts of ethanol were prepared for comparative purposes. 1ml of each of the above sterilising agent solutions and ethanol controls was added to individual samples of Dictol vaccine (24mls) to provide final sterilising agent concentrations of 0.4% w/v, 0.004% w/v and 0.00004% w/v for each sterilising agent and total ethanol concentrations of 4% w/v, 0.04% w/v and 0.0004% w/v in the ethanol controls. Viability of the Dictyocaulus viviparus third stage larvae was assessed at 3 hours and 6 days whilst sterility was assessed at 24 hours. The following results were obtained:

### Table 3

| Sterilant | | Viability | | Sterility |
|---|---|---|---|---|
| | | 3 hours | 6 days | 24 hours |
| 2,4 Dichlorophenol | 0.4% | dead | dead | sterile |
| | 0.004% | live | live | sterile |
| | 0.00004% | live | live | non-sterile |
| 4-Chloro-3-methylphenol | | | | |
| | 0.4% | dead | dead | sterile |
| | 0.004% | live | live | sterile |
| | 0.00004% | live | live | non-sterile |
| Chloramine T. | 0.4% | live | live | sterile |
| | 0.004% | live | live | sterile |
| | 0.00004% | live | live | non-sterile |
| Ethanol control | 4% | live | live | non-sterile |
| | 0.04% | live | live | non-sterile |
| | 0.0004% | live | live | non-sterile |

CLAIMS

1.    A vaccine for use in immunising cattle against <u>Dictyocaulus</u> <u>viviparus</u> infection which vaccine is a sterile composition comprising live immunogenic attenuated <u>Dictyocaulus</u> <u>viviparus</u> third stage larvae.

2.    A vaccine according to claim 1 wherein the <u>Dictyocaulus</u> <u>viviparus</u> larvae are in suspension in a sterile physiologically acceptable medium.

3.    A vaccine according to claim 1 or claim 2 wherein the <u>Dictyocaulus</u> <u>viviparus</u> larvae are sterilised with an effective concentration of a chemical sterilant capable of sterilising the larvae without causing exsheathment or impairment of the viability, infectivity and longevity of the larvae.

4.    A vaccine according to claim 3 wherein said chemical sterilant is an acid, an oxidising agent or a protein poison.

5.    A vaccine according to claim 3 wherein said chemical sterilant is a 2,2'-dihydroxy chlorinated diphenylmethane.

6.    A vaccine according to claim 5 wherein said chemical sterilant is 5,5'-dichloro-2,2'-dihydroxydiphenylmethane or a water soluble physiologically acceptable salt thereof.

7.    A vaccine according to claim 3 wherein said chemical sterilant is a halogenated phenol or a chloramine.

8.    A vaccine according to claim 7 wherein the sterilant is 2,4-dichlorophenol, 4-chloro-3-methylphenol or chloramine-T.

9.    A vaccine according to any one of claims 1 to 8 which is suitable for parenteral administration.

10.   A vaccine according to claim 9 in unit dosage form which comprises from 500 to 3000 sterile <u>Dictyocaulus</u> <u>viviparus</u> larvae in a sterile physiologically acceptable injection vehicle.

11.   A vaccine according to any one of claims 1 to 8 in unit dosage form for oral administration which comprises from 500 to 3000 sterile <u>Dictyocaulus</u> <u>viviparus</u> larvae in a sterile physiologically acceptable oral ingestion vehicle.

12. The use of a chemical sterilant according to any one of claims 3 to 8 for sterilisation of Dictyocaulus viviparus larvae without impairment of their viability.

13. A method of preparing a vaccine according to claim 1 which method comprises the steps of sterilising Dictyocaulus viviparus larvae before or after attenuation, without damaging their immunogenicity and presenting the third stage larvae in a sterile physiologically acceptable vehicle.

14. A method according to claim 13 which method comprises contacting said larvae with an effective concentration of a chemical sterilant which is capable of sterilising the larvae without causing exsheathment or impairment of the viability, infectivity and longevity of the larvae.

1. A method of preparing a vaccine for use in immunising cattle against <u>Dictyocaulus viviparus</u> infection which vaccine is a sterile composition comprising live immunogenic attenuated <u>Dictyocaulus viviparus</u> third stage larvae, which method comprises the steps of sterilising <u>Dictyocaulus viviparus</u> larvae before or after attenuation, without damaging their immunogenicity.

2. A method according to claim 1 which includes the step of presenting the third stage larvae in a sterile physiologically acceptable vehicle.

3. A method according to claim 1 or claim 2 wherein the larvae are sterilised in an aqueous medium containing an effective concentration of a chemical sterilant which is capable of sterilising the larvae without causing exsheathment or impairment of the viability, infectivity and longevity of the larvae.

4. A method according to claim 3 wherein said chemical sterilant is an acid, an oxidising agent, or a protein poison.

5. A method according to claim 3 wherein said chemical sterilant is a 2,2'-dihydroxy chlorinated diphenylmethane.

6. A method according to claim 5 wherein said chemical sterilant is 5,5'-dichloro-2,2'-dihydroxydiphenylmethane or a water soluble physiologically acceptable salt thereof.

7. A method according to claim 3 wherein said chemical sterilant is a halogenated phenol or a chloramine.

8. A method according to claim 7 wherein the sterilant is 2,4-dichlorophenol, 4-chloro-3-methylphenol or chloramine-T.

9. A method according to any one of claims 1 to 8 wherein the larvae are presented in a sterile medium suitable for parenteral administration.

10. A method according to claim 9 wherein the vaccine is presented in unit dosage form which comprises from 500 to 3000 sterile <u>Dictyocaulus viviparus</u> larvae in a sterile physiologically acceptable injection vehicle.

11. A method according to any one of claims 1 to 8 wherein the vaccine is presented in unit dosage form for oral administration which comprises from 500 to 3000 sterile Dictyocaulus viviparus larvae in a sterile physiologically acceptable oral ingestion vehicle.

12. The use of a chemical sterilant according to any one of claims 3 to 8 for sterilisation of Dictyocaulus viviparus larvae without impairment of their viability.

13. A method according to any one of claims 1 to 11 which includes the preliminary step of collecting Dictyocaulus viviparus larvae from infected bovine faeces.